(19) Europäisches Patentamt
European Patent Office
Office européen des brevets

(11) EP 2 746 751 A1

(12) EUROPÄISCHE PATENTANMELDUNG

(43) Veröffentlichungstag:
25.06.2014 Patentblatt 2014/26

(51) Int Cl.:
G01N 21/85 (2006.01)   G01N 33/18 (2006.01)
G01N 21/15 (2006.01)   G01N 21/27 (2006.01)

(21) Anmeldenummer: 13196915.6

(22) Anmeldetag: 12.12.2013

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(30) Priorität: 18.12.2012   AT 505982012
18.12.2012   DE 102012112541

(71) Anmelder: Zentrum für biomedizinische
Technologie der Donau-
Universität Krems
3500 Krems (AT)

(72) Erfinder:
• Brandl, Martin
3620 Spitz (AT)
• Straub, Andrea
03130 Felixsee (DE)
• Koch, Roland
02953 Bad Muskau (DE)
• Kellner, Karlheinz
3504 Krems-Stein (AT)
• Posnicek, Thomas
3485 Haitzendorf (AT)

(74) Vertreter: Patentanwaltskanzlei
Matschnig & Forsthuber OG
Siebensterngasse 54
1070 Wien (AT)

(54) Vorrichtung zur optischen Überwachung eines Parameters einer Flüssigkeitsprobe

(57) Vorrichtung (100) zur Überwachung von Parametern einer wässrigen Flüssigkeit, insbesondere Abwasser, aufweisend eine Messkammer (121) mit einer Zuleitung (101) für die zu untersuchende Flüssigkeit und einer Ableitung (105), eine optische Messanordnung mit zumindest einem optischen Sensor (104a, 104b) und eine Steuer- und Auswerteeinrichtung (112), wobei die optische Messanordnung einen ersten und einen zweiten optischen Sensor (104a, 104b) aufweist, wobei der erste optische Sensor (104a) eine UV-Lichtquelle (122) aufweist, wobei der zweite optische Sensor (104b) eine Lichtquelle (125), die sichtbares Licht emittiert aufweist, wobei die Zuleitung (101) oder die Ableitung (105) zumindest ein Ventilmittel (107) aufweist, durch welches zumindest ein Reinigungsfluid in die Messkammer (121) zuführbar ist, die Vorrichtung (100) zumindest ein Pumpmittel (103) zum Leiten der Flüssigkeit bzw. des zumindest einen Reinigungsfluids durch die Messkammer (121) aufweist, und die Steuer- und Auswerteeinrichtung (112) zur programmierbaren Steuerung zumindest der Pump- und Ventilmittel (103, 107, 108) und zum Auswerten der mittels der optischen Messanordnung ermittelten Messwerte eingerichtet ist, wobei die Steuer- und Auswerteeinrichtung (112) dazu eingerichtet ist, aufgrund der mit Reinigungsfluid befüllten Messkammer (121) Referenzmessungen durchzuführen. Die Erfindung betrifft ferner ein Verfahren zum Überwachen von Parametern einer wässrigen Flüssigkeit unter Verwendung einer solchen Vorrichtung (100).

Fig. 1

**EP 2 746 751 A1**

**Beschreibung**

Die Erfindung betrifft eine Vorrichtung zur Überwachung von Parametern einer wässrigen Flüssigkeit, insbesondere Abwasser, aufweisend eine Messkammer mit einer Zuleitung für die zu untersuchende Flüssigkeit und einer Ableitung, eine optische Messanordnung mit zumindest einem optischen Sensor zur Messung zumindest einer optischen Eigenschaft der Flüssigkeit, und eine Steuer- und Auswerteeinrichtung, wobei die optische Messanordnung einen ersten optischen Sensor und einen zweiten optischen Sensor aufweist, wobei der erste optische Sensor eine erste Lichtquelle, die UV-Licht emittiert, einen ersten Photodetektor und einen zwischen der ersten Lichtquelle und dem ersten Photodetektor verlaufenden ersten Messpfad aufweist, wobei der zweite optische Sensor eine zweite Lichtquelle, die sichtbares Licht emittiert, einen zweiten Photodetektor und einen zwischen der zweiten Lichtquelle und dem zweiten Photodetektor verlaufenden zweiten Messpfad aufweist, wobei der erste und der zweite Messpfad durch die Messkammer und die darin befindliche Flüssigkeit verlaufen, die Zuleitung oder die Ableitung zumindest ein Ventilmittel aufweist, durch welches zumindest ein Reinigungsfluid in die Messkammer zuführbar ist, die Vorrichtung zumindest ein Pumpmittel zum Leiten der Flüssigkeit bzw. des zumindest einen Reinigungsfluids durch die Messkammer aufweist, und die Steuer- und Auswerteeinrichtung zur programmierbaren Steuerung zumindest der Pump- und Ventilmittel und zum Auswerten der mittels der optischen Messanordnung ermittelten Messwerte eingerichtet ist. Die Erfindung betrifft ferner ein Verfahren zum Überwachen von Parametern einer wässrigen Flüssigkeit, insbesondere Abwasser, unter Verwendung einer solchen Vorrichtung.

Für die Betriebssicherheit, die Steuerung technologischer Abläufe und die Wartungseffizienz wasserwirtschaftlicher Aufbereitungsanlagen ist es von großer Bedeutung, den Klärprozess anhand von Qualitätsparametern, die den Verschmutzungsgrad von Abwässern charakterisieren, in ausreichend kurzen, periodischen Zeitabständen zu überwachen. Auf diese Weise kann überprüft werden, ob eine Anlage einwandfrei arbeitet. Fehlfunktionen der Anlage können zu einer unzureichenden Klärung von Abwässern führen, welche wiederum die chemische, biologische und ökologische Qualität von Gewässern, in welche das Abwasser nach Verlassen der Aufbereitungsanlage eingeleitet wird, beeinträchtigen kann. Aus diesem Grund ist es in vielen Staaten im Sinne einer nachhaltigen und umweltverträglichen Wassernutzung gesetzlich vorgeschrieben, bei der Abwasserreinigung einen bestimmten technischen Standard, Wartungsregeln und Grenzwerte einzuhalten.

Mittels der Spektralphotometrie lassen sich photometrische Messwerte von Abwässern direkt ermitteln. Diese Messwerte können anschließend mit Hilfe mathematischer Auswerteverfahren in spezifische Abwasserparameter, die Rückschluss auf die Funktionsfähigkeit der Aufbereitungsanlage geben, umgerechnet werden. Aus der Messung des spektralen Absorptionskoeffizienten mit UV-Licht bei einer Wellenlänge von 254 nm (SAK 254 [1/m]) lassen sich der CSB (chemische Sauerstoffbedarf) und der BSB (biologischer Sauerstoffbedarf) ableiten, die als wichtigste Parameter in der Abwasseranalytik gelten. Der CSB ist ein Bewertungsparameter für die Gesamtbelastung von Fließgewässern und Abwässern mit organischen Verbindungen, wobei auch biologisch schwer oder nicht abbaubare Verbindungen inbegriffen sind. Der BSB ist eine relative Kenngröße für die Menge an Sauerstoff, die beim oxidativen Abbau von Wasserinhaltsstoffen durch die im Abwasser vorhandenen Mikroorganismen innerhalb einer bestimmten Zeit, üblicherweise 5 Stunden, verbraucht wird. Der BSB ist somit eine Kenngröße für biologisch abbaubare organische Substanzen. Darüber hinaus kann die Färbung des Wassers so wie der DOC (gelöster organisch gebundener Kohlenstoff) anhand des spektralen Absorptionskoeffizienten bei einer Wellenlänge von 436 nm (SAK 436 [1/m]) charakterisiert werden, der auch Rückschluss auf die Huminstoffbelastung gibt. Der Zusammenhang zwischen DOC und Huminstoffen ist einem Fachmann hinlänglich bekannt. Der Vorteil der photometrischen Abwasseranalyse liegt insbesondere darin, dass das Messverfahren im Wesentlichen ohne Reagenzien auskommt und trotzdem zuverlässige Messwerte liefert.

Vor allem in dünn besiedelte Regionen kommen zur Abwasserreinigung dezentrale Kleinkläranlagen zum Einsatz, da diese Regionen aus technischen oder finanziellen Gründen oftmals nicht an große kommunale Wasseraufbereitungsanlagen angeschlossen werden können. Kleinkläranlagen erfordern behördlich vorgeschriebene Kontrollen, um sicherzustellen, dass die Anlage einwandfrei arbeitet, sowie die Einhaltung von Wartungsregeln. Die Kontrollen werden meistens vom Betreiber selbst durchgeführt, da eine regelmäßige Begutachtung durch Wartungs- und Serviceunternehmen aufgrund der langen Anfahrtswege mit laufenden Kosten verbunden ist.

Aus diesem Grund wurden Kontrollvorrichtungen entwickelt, die auf dem Prinzip der Spektralphotometrie basieren, und mit welchen wichtige Kenngrößen der Abwasseranalytik durch den Betreiber selbst laufend ermittelt und überwacht werden können.

Aus der DE 10 2009 028 254 A1 ist eine Vorrichtung bekannt geworden, mit der Abwasserparameter wie z.B. Nitrat oder spektrale Absorptionskoeffizienten spektralphotometrisch ermittelt werden, indem ein Lichtstrahl durch eine Messkammer geführt und detektiert wird. Zum Durchführen der Referenzmessung wird ein Referenzabsorber in Form eines Festkörpers in die Messkammer eingeführt. Die Reinigung der Komponenten ist relativ umständlich, da nicht nur die Messkammer, sondern auch der Referenzabsorber regelmäßig gereinigt werden muss, um eine Biofilmbildung zu vermeiden und um aussagekräftige Messwerte zu generieren.

Die GB 2 256 043 A offenbart eine Kontrollvorrichtung, die einen Kanal einschließt, durch welchen die zu untersuchende Flüssigkeit hindurchströmt und eine Mehrzahl entlang des Kanals angeordneter Sensoren, einschließlich optischer Sensoren, zum Messen der Flüssigkeitseigenschaften. Aufgrund der im Kanal auftretenden Flüssigkeitsturbulenzen kann es leichter zu einer Verschmutzung ("fouling") der Kanalinnenwände kommen. Um die damit einhergehende Sensordrift zu kompensieren, wird darin vorgeschlagen, den Sensor in periodischen Intervallen zu rekalibrieren. Eine Referenzmessung wird nicht durchgeführt, wodurch sich die Notwendigkeit einer aufwendigen Temperaturkompensation ergibt. Die Reinigung der Vorrichtung erfolgt manuell.

Die EP 0 724 718 beschreibt eine Vorrichtung zum Messen von Eigenschaften einer Flüssigkeit, wobei die Vorrichtung einen Kanal einschließt, durch welchen die Flüssigkeit hindurchströmt und eine Mehrzahl entlang des Kanals angeordneter Sensoren, einschließlich optischer Sensoren, zum Messen der Flüssigkeitseigenschaften. Die Querschnittsform des Kanals ändert sich in Abhängigkeit des jeweils angeordneten Sensors. Für die Reinigung des Kanals ist ein apparativ aufwändiger Reinigungsmechanismus in Form eines sich längs des Kanals bewegenden Sprühkopfs vorgesehen.

Die DE 102 28 929 A1 offenbart eine Vorrichtung zur photometrischen Messung des Gehalts einer chemischen Substanz in einer Messlösung, insbesondere zur Messung von Nitrat und gelösten organischen Substanzen. Die Vorrichtung weist als Lichtquelle eine Blitzlampe sowie zwei Empfangseinheiten auf, welche die durch die Messlösung transmittierte Strahlung empfangen. Das Licht der Blitzlampe wird in Abhängigkeit der gemessenen Intensitätswerte geregelt.

In der DE 10 2008 028 058 A1 ist ein Trübungsmessgerät für den Auslauf von Kläranlagen beschrieben, das einen optischen Sensor zur Messung der Trübung im Infrarot-Bereich aufweist. Das Gerät weist ferner UV-LEDs auf, um der Entstehung von Biofilmen in der Messstrecke des Trübungsmessgeräts mittels UV-Licht entgegenzuwirken.

Die DE 10 2004 063 720 beschreibt eine Kontrollvorrichtung für Abwasser, die einen Sensor, durch den ein Qualitätsparameter des Abwassers ermittelbar und als elektronisches Signal übertragbar ist, aufweist.

Die DE 69227764 T2 beschreibt eine Überwachungsvorrichtung wie eingangs genannt zum Messen von organischen Verunreinigungen in einer Flüssigkeit. Der DE 69107551 T2 ist eine Vorrichtung und ein Verfahren zum Überwachen von Parametern von flüssigen Proben zu entnehmen. Die EP 1962089 B1 offenbart eine Einmalsonde für Temperatur- und Fluoreszenzmessungen in Unterseeumgebung.

Die bekannten Vorrichtungen zeichnen sich durch einen verhältnismäßig hohen apparativen Aufwand, einen komplexen Messablauf und einen umständlichen Reinigungsmechanismus aus und erfordern kurze Wartungsintervalle, wodurch sich erhebliche Nachteile in Bezug auf deren Mobilität, deren Kosten und deren Benutzerfreundlichkeit ergeben. Zusätzlich zu den genannten Nachteilen sind die derzeit am Markt erhältlichen Kontrollvorrichtungen für Kleinkläranlagenbetreiber sehr teuer in der Anschaffung.

Es besteht daher ein Bedarf an einer kostengünstigen, einfach zu bedienenden und zu wartenden Vorrichtung zur Überwachung von Parametern einer wässrigen Flüssigkeit, insbesondere Abwasser. Der Vorrichtung soll ein einfaches Messverfahren zugrunde liegen und es insbesondere einem Betreiber einer Kleinkläranlage ermöglichen, die vorgeschriebenen Kontrollen effizient durchzuführen und vorhandene Funktionsprobleme gezielt, mit geringen Kosten und kurzfristig zu beheben. Es ist daher eine Aufgabe der Erfindung diesen Bedarf zu decken.

Diese Aufgabe wird durch eine Vorrichtung wie eingangs genannt gelöst, welche erfindungsgemäß dadurch gekennzeichnet ist, dass die Steuer- und Auswerteeinrichtung dazu eingerichtet ist, aufgrund der mit Reinigungsfluid befüllten Messkammer Referenzmessungen durchzuführen.

Dank der Erfindung wird eine Überwachungsvorrichtung bereitgestellt, mit welcher Parameter einer wässrigen Flüssigkeit, insbesondere Abwasser, benutzerfreundlich sowie wartungs- und kosteneffizient bestimmt werden können, so dass der sichere Betrieb wasserwirtschaftlicher Aufbereitungsanlagen, insbesondere Kleinkläranlagen, und die einwandfreie Steuerung der darin stattfindenden technologischen Abläufe zuverlässig gewährleistet wird. Die Erfindung erleichtert zudem die gesetzeskonforme Umsetzung gesetzlicher Vorgaben, besonders für Betreiber von Kleinkläranlagen. Für Österreich geltende Rechtsquellen sind nach derzeitigem Stand beispielsweise die EU-Wasserrahmenrichtlinie 2000/60/EG, das Wasserrechtsgesetz und die österreichische Trinkwasserverordnung.

Für Referenzmessungen zu Kalibrierzwecken bzw. um Temperatureinflüsse, die durch die Lichtquellen bedingt sind, zu kompensieren, ist die Steuer- und Auswerteeinrichtung erfindungsgemäß dazu eingerichtet, aufgrund der mit Reinigungsfluid befüllten Messkammer Referenzmessungen durchzuführen. Dabei wird das Reinigungsfluid direkt als Referenzflüssigkeit verwendet und ermöglicht dadurch einen sehr einfachen und kostengünstigen apparativen Aufbau der erfindungsgemäßen Vorrichtung. Um eine der zum Anmeldezeitpunkt geltenden DIN-Norm Nr. 38404-3/Juli 2005 entsprechende Nullmessung durchführen zu können, ist es zweckmäßig, wenn als Reinigungsfluid und somit als Referenzflüssigkeit optisch reines Wasser verwendet wird. Mit Vorteil handelt es sich dabei um optisch reines Wasser der Qualität 1 nach DIN ISO 3696 (Wasser für analytische Zwecke), welches beispielsweise hergestellt wird, indem ein Membranfilter der Porenweite 0,1 μm für etwa 1 h in destilliertem oder deionisiertem Wasser eingeweicht wird und anschließend etwa 1 Liter destilliertes oder deionisiertes Wasser durch das vorbereitete Filter filtriert wird.

Der Begriff "wässrige Flüssigkeit" wie hierin verwendet, bezieht sich in erster Linie auf alle Arten von Wasser wie Abwasser, Fluss- und Seewasser, technische Prozesswässer sowie Trinkwasser, Mineralwasser und Wasser, welches zur Trinkwassergewinnung bereitgestellt wird. Insbesondere bezieht sich der Begriff jedoch auf Abwasser, wobei der Begriff "Abwasser" auch gereinigtes Abwasser bzw. geklärtes Abwasser (Klarwasser) einschließt.

Der Begriff "Reinigungsfluid" bezieht sich auf Reagenzien und Reinigungsmittel, die zum Vermeiden einer Biofilmbildung in der Messkammer sowie in deren Zu- und Ableitungen geeignet sind. Das Reinigungsfluid kann beispielsweise Wasser, vorzugsweise optisch reines Wasser, destilliertes Wasser, ionenfreies Wasser, keimfreies Wasser, ein Desinfektionsmittel, Alkohol oder eine Osmoselösung, z.B. Osmosewasser mit einem Leitwert von < 20 μS, sein.

Die hierin verwendeten Begriffe Pumpmittel (Pumpe) bzw. Ventilmittel (Ventil) beziehen sich auf Pumpen bzw. Ventile, die für den Einsatz in einem, eine Flüssigkeit führenden Leitungssystem geeignet und als dem Fachmann notorisch bekannt vorauszusetzen sind. Das Pumpmittel/die Pumpe kann beispielsweise eine Membranpumpe sein. Das Ventilmittel/das Ventil kann beispielsweise als Elektromagnetventil oder als pneumatisches Ventil realisiert sein. Die Pump- und Ventilmittel sind von der Steuer- und Auswerteeinrichtung nach an sich bekannter Art steuerbar.

Das Ventilmittel (Ventil) zum Zuführen des Reinigungsfluids ist vorzugsweise in der Zuleitung angeordnet. Das Reinigungsfluid (gegebenenfalls auch mehrere verschiedene Reinigungsfluida) befindet sich in einem wiederbefüllbaren Reinigungsbehälter für das Reinigungsfluid und kann mittels des zumindest einen Pumpmittels (Pumpe) über das Ventilmittel aus dem Reinigungsbehälter in die Zuleitung und weiter durch die Messkammer sowie die Ableitung geführt werden. Um den Aufbau der Vorrichtung so einfach wie möglich zu gestalten, ist es von Vorteil, wenn nur eine Art von Reinigungsfluid vorgesehen ist.

Ist das Ventilmittel in der Ableitung angeordnet, dann wird das Reinigungsfluid aus dem wiederbefüllbaren Reinigungsbehälter mittels des Pumpmittels über das Ventilmittel in die Ableitung und weiter durch die Messkammer in die Zuleitung geführt.

Die Vorrichtung ist normalerweise in unmittelbarer Nähe zur Probeentnahmestelle für die zu untersuchende wässrige Flüssigkeit stationär verankert, beispielsweise im Wasserzulauf oder Wasserablauf einer wasserwirtschaftlichen Anlage. Die Vorrichtung kann beispielsweise mit Hilfe von Kabelbindern oder dergleichen an vorhandenen Bauelementen der wasserwirtschaftlichen Anlage fixiert werden. Die Zuleitung und gegebenenfalls auch die Ableitung tauchen dabei in den Wasserkörper, aus welchem die zu untersuchende Flüssigkeitsprobe entnommen wird, ein. Vorzugsweise ist die Probeneinlassöffnung der Zuleitung in einem definierten Abstand zur Wasseroberfläche angeordnet. Zum Einhalten dieses Abstands kann an der Zuleitung ein Schwimmkörper (Schwimmer) befestigt sein.

Für die Bestimmung des spektralen Absorptionskoeffizienten bei einer Wellenlänge von 254 nm (SAK 254) von Abwasser, welche mit Vorteil in Entsprechung mit dem deutschen Einheitsverfahren zur Wasser-, Abwasser- und Schlammuntersuchung (DIN 38404-3; derzeitiger Stand: Juli 2005) durchgeführt wird, ist es zweckmäßig, wenn die erste Lichtquelle UV-Licht mit einer Wellenlänge von 254 nm und die zweite Lichtquelle sichtbares Licht mit einer Wellenlänge von 550 nm emittiert. Bei dieser Ausführungsform wird die sich in der Messkammer befindliche Flüssigkeit folglich mit UV-Licht bei 254 nm und mit sichtbaren Licht bei 550 nm (zur Kompensation von ungelösten Wasserinhaltsstoffen) durchleuchtet und die transmittierte Lichtintensität mittels der Photodetektoren gemessen und in ein elektrisches Signal umgewandelt. Aus den erhaltenen Intensitätswerten wird nach an sich bekannter Art und Weise der SAK 254 berechnet, aus welchem wiederum der CSB und der BSB ableitbar sind. Die Auswertung der Messergebnisse erfolgt erfindungsgemäß durch die Steuer- und Auswerteeinrichtung und ist weiter unten in den Beispielen näher ausgeführt. Für die Bestimmung des SAK 254 von Trinkwasser ist die Messung bei 550 nm im Normalfall nicht notwendig, da Trinkwasser typischerweise keine ungelösten Bestandteile enthält. In diesem Fall kann die Steuer- und Auswerteeinheit so programmiert sein, dass nur die Messung bei 254 nm durchgeführt wird und die Messung bei 550 nm entfällt.

Eine vorteilhafte Weiterbildung der Erfindung sieht vor, dass die optische Messanordnung einen dritten optischen Sensor aufweist, der eine dritte Lichtquelle, die sichtbares Licht emittiert, einen dritten Photodetektor und einen zwischen der dritten Lichtquelle und dem dritten Photodetektor verlaufenden dritten Messpfad aufweist, wobei der dritte Messpfad durch die Messkammer und die darin befindliche Flüssigkeit verläuft. Für die Bestimmung der Färbung der Flüssigkeit, die anhand des spektralen Absorptionskoeffizienten bei einer Wellenlänge von 436 nm (SAK 436 in [1/m]) charakterisiert wird, ist es daher zweckmäßig, wenn die dritte Lichtquelle sichtbares Licht mit einer Wellenlänge von 436 nm emittiert. Der SAK 436 kann darüber hinaus als Kenngröße für DOC und damit auch für die Huminstoffbelastung herangezogen werden.

Die Vorrichtung lässt sich besonders leicht realisieren, wenn die erste, die zweite und die dritte Lichtquelle eine wellenlängenspezifische LED (Light Emitting Diode) aufweisen. LEDs haben ferner den Vorteil einer längeren Haltbarkeit als die ansonsten häufig in der Photometrie eingesetzten Blitzlampen. Bei einer bevorzugten Ausführungsform emittiert die LED der ersten Lichtquelle UV-Licht einer Wellenlänge von 254 nm und die LED der zweiten Lichtquelle sichtbares Licht einer Wellenlänge von 550 nm. Ist zudem ein dritter optischer Sensor vorgesehen, dann ist es von Vorteil, wenn die LED der ersten Lichtquelle UV-Licht einer Wellenlänge von 254 nm, die LED der zweiten Lichtquelle sichtbares Licht einer Wellenlänge von 550 nm und die LED der dritten Lichtquelle sichtbares Licht einer Wellenlänge von 436 nm emittiert.

Alternativ zur UV-LED kann als erste Lichtquelle auch eine UV-Niederdrucklampe (z.B. von Heraeus Noblelight) zur Anwendung kommen.

Um Schwankungen der UV-Licht-Intensität der ersten Lichtquelle zu kompensieren, ist bei einer ersten vorteilhaften Weiterbildung zwischen der ersten Lichtquelle und der Messkammer ein Strahlteiler angeordnet, welcher einen Anteil des von der ersten Lichtquelle emittierten UV-Lichts in einen Referenzmesspfad abzweigt und einem Referenzphotodetektor zuführt. Der Strahlteiler ist vorzugsweise als halbdurchlässiger Spiegel realisiert. Beispielsweise werden durch den Strahlteiler ungefähr 30% des Lichts für die Referenzmessung in einen Referenzmesspfad abgezweigt und einem Referenzdetektor zugeführt.

Alternativ zur zuvor genannten Weiterbildung, die einen Strahlteiler vorsieht, können bei einer anderen vorteilhaften Weiterbildung auftretende Schwankungen der UV-Licht-Intensität der ersten Lichtquelle mit Vorteil kompensiert werden, wenn die Steuer- und Auswerteeinheit dazu eingerichtet ist, die Schwellspannung des ersten Photodetektors zu erfassen und bei der Auswertung der Messwerte zu berücksichtigen.

Die ersten, zweiten und dritten Photodetektoren sowie der Referenzdetektor können jeweils ein photoelektrisches Element, insbesondere eine Photodiode, umfassen, welches ein der detektierten Lichtintensität entsprechendes elektrisches Signal generiert und dieses der Steuer-und Auswerteeinheit zuführt. Mit Vorteil weisen die eingesetzten Photodetektoren eine möglichst geringe Bandbreite auf oder sind nur für die entsprechende Wellenlänge empfindlich. Beispielsweise können der erste Photodetektor und der Referenzdetektor vom Typ BPW21 (Siemens), der zweite Photodetektor vom Typ TW30SX (Texas Instruments) und der dritte Photodetektor vom Typ OSD15-5T (OSI Optoelectronics) sein. Den genannten Detektoren kann ferner nach bekannter Art und Weise ein Vorverstärker zur Verstärkung des elektrischen Signals nachgeschaltet sein.

Um aufgrund einer zu großen Bandbreite auftretende Fehlmessungen ausschließen zu können, ist es von Vorteil, wenn im ersten Messpfad an einer Position zwischen der ersten Lichtquelle und dem ersten Photodetektor ein Bandpassfilter angeordnet ist. Die Bandbreite ist von den eingesetzten optischen Bauelementen abhängig und ergibt sich aus der Intensitätskennlinie der ersten Lichtquelle, der Transmissionskennlinie der Messkammer und des gegebenenfalls vorhandenen Strahlteilers sowie der Empfindlichkeitskennlinie des ersten Photodetektors. Obwohl Bauelemente bevorzugt werden, die eine geringe Bandbreite ergeben, ist es dank dieser Weiterbildung auch möglich, Bauteile zu verwenden, die eine größere Bandbreite mit sich bringen.

Um die gesetzlich vorgeschriebenen Betreiberkontrollen zu erleichtern, ist es von Vorteil, wenn die Vorrichtung eine Übertragungseinheit, z.B. ein GSM-Modul, zum Übertragen der ermittelten und/oder ausgewerteten Messwerte an einen externen Empfänger aufweist. Durch Übertragen der erhaltenen Messdaten an einen beliebigen Ort ist eine online-Fernüberwachung der Vorrichtung z.B. per Mobilfunk/GSM möglich und erspart die laufend durchzuführende Sichtkontrolle vor Ort. Etwaig vorhandene Funktionsprobleme können durch den Betreiber selbst oder ein Serviceunternehmen rasch erkannt und behoben werden. Der externe Empfänger ist beispielsweise ein Mobiltelefon oder ein Computer. Im Fall eines Mobiltelefons können die Messdaten zum Beispiel per SMS übertragen werden.

Die Vorrichtung kann ferner eine Bedienkonsole zur Eingabe von Befehlen für die Steuer-und Auswerteeinrichtung, beispielsweise zur Programmierung des Messablaufs und dessen zeitlichen Ablaufs, aufweisen. Die Bedienkonsole kann ferner ein Display zum Anzeigen der Befehle bzw. der Mess- und Kenndaten oder einen Touchscreen aufweisen.

Mit Vorteil weist die Vorrichtung zur Energieversorgung ein austauschbares Energieversorgungsmodul, beispielsweise handelsübliche Trockenbatterien, auf. Mit Vorteil ist der Vorrichtung zur Batteriestandskontrolle ferner eine Alarmeinrichtung zugeordnet, welche bei geringer oder einem Ausfall der Energieversorgung ein Alarmsignal auslöst. Die Alarmeinrichtung kann Bestandteil der Steuer- und Auswerteeinrichtung oder ein eigenes Bauteil sein. So kann bei einem Leistungsabfall oder einem Ausfall des Energieversorgungsmoduls, wodurch die Funktionssicherheit der Vorrichtung nicht mehr gewährleistet ist, ein akustisches Signal aktiviert, eine Anzeige am Display angezeigt oder mittels der Übertragungseinrichtung eine Information an den externen Empfänger, z.B. in Form eines SMS an ein Mobiltelefon, übermittelt werden.

Darüber hinaus kann mittels eines Zählers (Counter), der die Messzyklen zählt, der Füllstand des Reinigungsfluids überwacht werden, wobei in Analogie zur Batteriestandskontrolle ein Alarmsignal wie oben beschrieben übermittelt wird, wenn der Füllstand eine vorgegebene Grenze unterschreitet.

Der Vorrichtung kann ferner ein Temperaturfühler (Temperatursensor) zugeordnet sein, der sich vorzugsweise in der Nähe der Messkammer befindet und mit der Steuer- und Auswerteeinrichtung in Signalverbindung steht. Temperaturabweichungen, die über einen vorgebbaren Temperaturbereich hinausgehen, können ebenfalls als Alarmsignal wie oben beschrieben übermittelt werden.

Um denjenigen Abschnitt der Zuleitung zu reinigen, der stromauf des zumindest einen Ventilmittels zum Zuführen des Reinigungsfluids liegt, ist es von Vorteil, wenn das zumindest eine Ventilmittel in der Zuleitung angeordnet ist und die Vorrichtung ein Rückspülventil aufweist, welches in der Zuleitung an einer Position zwischen dem zumindest einen Ventilmittel und der Messkammer angeordnet ist und von welchem sich eine Rückspülleitung zur Zuleitung erstreckt, wobei die Rückspülleitung an einer Position stromauf des zumindest einen Ventilmittels in die Zuleitung mündet. Durch diese apparative Anordnung ist es möglich, die Zuleitung der Messkammer mit dem Reinigungsfluid durch Umkehren der Fließrichtung des Reinigungsfluids rückzuspülen und somit einer Biofilmbildung entgegenzuwirken.

Die Begriffe "stromauf" und "stromab" beziehen sich immer auf die Fließrichtung der zu untersuchenden wässrigen Flüssigkeit (= Wasserprobe), welche durch die Zuleitung in die Messkammer geführt wird und von dort weiter in die Ableitung fließt.

Um die Vorrichtungsbestandteile vor Spritzwasser zu schützen, ist diese mit Vorteil in einem wasserdichten Gehäuse untergebracht, wobei das Gehäuse Zu- und Ableitungsöffnungen aufweist, durch welche sich die Zu- und Ableitungen erstrecken.

Ein weiterer Gegenstand der Erfindung bezieht sich auf ein Verfahren zum Überwachen von Parametern einer wässrigen Flüssigkeit, insbesondere Abwasser, unter Verwendung einer Vorrichtung gemäß den beiliegenden Ansprüchen und wie oben beschrieben, wobei das Verfahren durch die folgenden Schritte gekennzeichnet ist:

a) Spülen der Messkammer mit der wässrigen Flüssigkeit,

b) Bestimmen der optischen Eigenschaften der wässrigen Flüssigkeit mittels des ersten optischen Sensors, und gegebenenfalls mittels des zweiten und/oder dritten optischen Sensors,

c) Spülen der Messkammer mit zumindest einem Reinigungsfluid,

d) Auswerten der mittels der optischen Sensoren erhaltenen Messwerte mittels der Steuer-und Auswerteeinrichtung, und

e) Übermitteln der ausgewerteten Messwerte mittels einer Übertragungseinheit, insbesondere eines GSM-Moduls, an einen externen Empfänger.

Wie weiter oben bereits beschrieben wurde, ist es für Referenzmessungen zu Kalibrierzwecken bzw. um Temperatureinflüsse, die durch die Lichtquellen bedingt sind, zu kompensieren, günstig, wenn vor dem Bestimmen der optischen Eigenschaften der wässrigen Flüssigkeit eine Referenzmessung durchgeführt wird. Erfindungsgemäß wird deshalb vor Schritt a) mittels der ersten und zweiten optischen Sensoren, und gegebenenfalls mittels des dritten optischen Sensors, zumindest eine Referenzmessung durch Bestimmen der optischen Eigenschaften des sich in der Messkammer befindlichen Reinigungsfluids durchgeführt. Somit wird das Reinigungsfluid direkt als Referenzflüssigkeit verwendet und ermöglicht dadurch einen sehr einfachen und kostengünstigen apparativen Aufbau der erfindungsgemäßen Vorrichtung. Um eine der derzeit geltenden DIN-Norm Nr. 38404-3/Juli 2005 entsprechende Nullmessung durchführen zu können, ist es zweckmäßig, wenn als Reinigungsfluid bzw. als Referenzflüssigkeit optisch reines Wasser wie weiter oben beschrieben verwendet wird.

Um eine Biofilmbildung in der Zuleitung zu vermeiden, ist es von Vorteil, wenn anschließend an Schritt c) die Zuleitung der Messkammer mittels zumindest eines Reinigungsfluids rückgespült wird.

Zur Bestimmung spezifischer Wasserparameter (z.B. CSB, BSB, DOC, Färbung/Huminstoffbelastung) ist es von Vorteil, wenn die Messungen mittels des ersten optischen Sensors bei einer Wellenlänge von 254 nm, mittels des zweiten optischen Sensors bei einer Wellenlänge von 550 nm und gegebenenfalls mittels des dritten optischen Sensors bei einer Wellenlänge von 436 nm durchgeführt werden.

Im Folgenden wird die Erfindung samt weiteren Vorzügen anhand eines nicht einschränkenden Ausführungsbeispiels erläutert, das in den beigefügten Zeichnungen dargestellt ist. Die Zeichnungen zeigen:

Fig. 1 eine schematische Blockdarstellung einer erfindungsgemäßen Vorrichtung,

Fig. 2 eine vergrößerte schematische Schnittdarstellung der Messeinrichtung aus der Fig. 1(in Aufsicht),

Fig. 3 eine weitere vergrößerte schematische Schnittdarstellung der Messeinrichtung aus der Fig. 1 (in Seitenansicht),

Fig. 4 bis 7 die Schritte eines Messablaufs anhand der Darstellung der wesentlichen Vorrichtungskomponenten aus der Fig. 1, und

Fig. 8 ein Ablaufdiagramm des Messablaufs samt Menüstruktur für die Befehlseingabe.

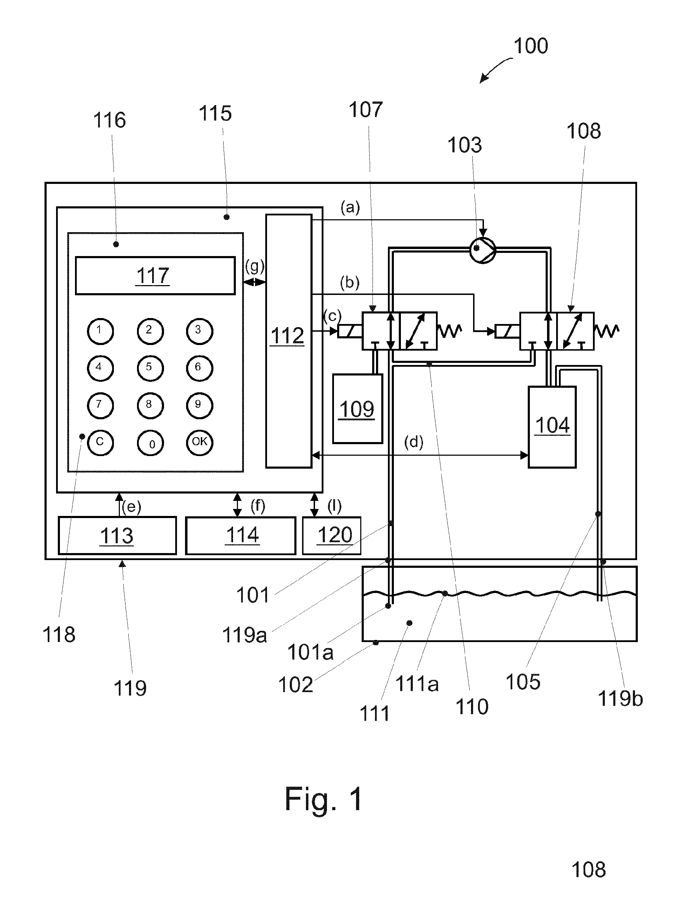

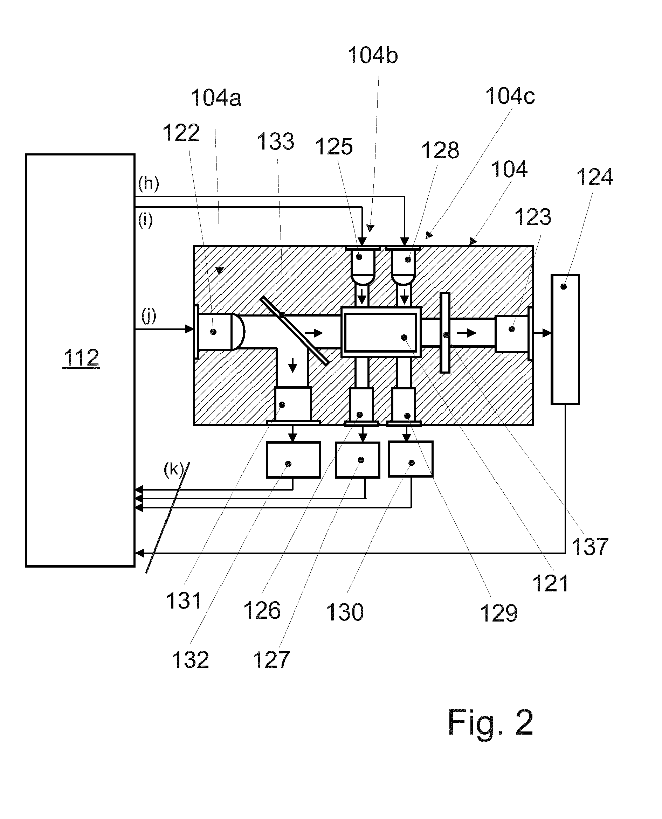

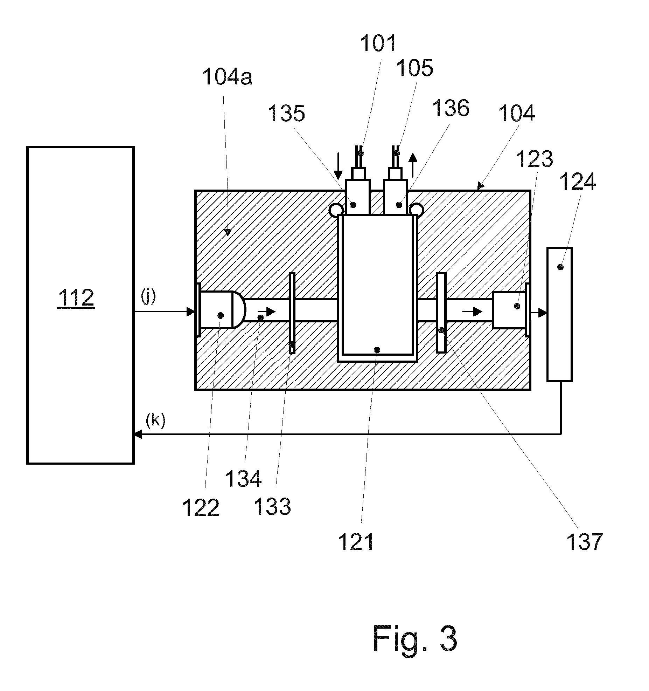

Die Fig. 1 zeigt eine schematische Blockdarstellung einer erfindungsgemäßen Vorrichtung 100 zum Überwachen von Parametern einer Wasserprobe aus einer wasserwirtschaftlichen Anlage. Über eine Zuleitung 101 wird aus einer Wasserleitung 102 eine Wasserprobe aus einem Wasserkörper 111, z.B. Trinkwasser oder Abwasser, mittels einer elektrisch betriebenen Membranpumpe 103 angesaugt und in eine, einer optischen Messeinrichtung 104 zugeordneten Messkammer 121 (siehe Detailansicht in Fig. 2 und 3) und von dort über eine Ableitung 105 wieder zurück in die Wasserleitung 102 befördert. Die Zuleitung 101 und die Ableitung 105 tauchen stationär in den Wasserkörper 111 der Wasserleitung 102 ein und sind dort verankert. Die Wasserleitung 102 ist insbesondere eine Abwasserleitung, kann aber auch eine Trinkwasserleitung sein. Die Wasserprobe gelangt über eine Probeneinlassöffnung 101a in die Zuleitung 101. Die Probeneinlassöffnung ist im Wasserkörper 111 vorzugsweise in einem definierten Abstand zur Wasseroberfläche 111a angeordnet. Zum Einhalten dieses Abstands kann an der Zuleitung 101 nach an sich bekannter Art ein nicht dargestellter Schwimmkörper (Schwimmer) befestigt sein.

Die optische Messeinrichtung 104 umfasst ferner eine optische Messanordnung bestehend aus mehreren optischen Sensoren, die an der Messkammer 121 angeordnet sind (optische Sensoren 104a, 104b, 104c; Detailansicht in Fig. 2 und 3 und Beschreibung hierzu weiter unten). Die Membranpumpe 103 ist in der Zuleitung 101 angeordnet. Ferner sind in der Zuleitung 101 ein Ansaugventil 107 und ein Rückspülventil 108 angeordnet. Das Ansaugventil 107 befindet sich stromauf der Membranpumpe 103, d.h. in einem stromauf der Membranpumpe 103 liegenden Abschnitt der Zuleitung 101. Das Rückspülventil 108 befindet sich in einem Abschnitt der Zuleitung 101 zwischen der Membranpumpe 103 und der Messeinrichtung 104. Die Vorrichtung 100 weist ferner einen Reinigungsbehälter 109 für eine Reinigungsflüssigkeit auf, welche aus dem Reinigungsbehälter 109 über das Ansaugventil 107 in die Zuleitung 101 und von dort weiter in die Messkammer 121 und die Ableitung 105 geleitet werden kann. Die Vorrichtung 100 weist darüber hinaus noch eine Rückspülleitung 110 auf, welche sich vom Rückspülventil 108 in die Zuleitung 101 erstreckt und an einer Position stromauf des Ansaugventils 107 in die Zuleitung 101 mündet.

Die Membranpumpe 103, das Ansaugventil 107, das Rückspülventil 108 und die optische Messeinrichtung 104 sind über Steuer- und Signalleitungen (a), (b), (c) und (d) mit einer Steuer- und Auswerteeinrichtung 112 zur Messablaufsteuerung und zur Messdatenauswertung verbunden, die in diesem Beispiel auf einer Hauptplatine 115 befestigt ist. Das Ansaugventil 107 und das Rückspülventil 108 können beispielsweise als gesteuerte 3/2-Wege-Elektromagnetventile oder als gesteuerte pneumatische Ventile realisiert sein und sind von der Steuer- und Auswerteeinrichtung 112 angesteuert. Die weiter unten im Detail beschriebenen optischen Sensoren 104a, 104b, 104c sind von der Steuer- und Auswerteeinrichtung 112 unabhängig voneinander aktivierbar. Die Steuer- und Auswerteeinrichtung 112 ist nach an sich bekannter Art aufgebaut und weist typischerweise einen Mikrokontroller sowie elektronische Bauteile auf.

Der Vorrichtung 100 ist darüber hinaus eine Übertragungseinheit 114 zur Fernübertragung von Daten zugeordnet, welche bei dem in der Fig. 1 gezeigten Beispiel ein GSM-Modul ist. Mit der Übertragungseinheit 114 werden die ermittelten und/oder ausgewerteten Messwerte an einen externen Empfänger (nicht dargestellt) übermittelt, der im vorliegenden Beispiel ein Mobiltelefon oder ein Computer ist. Beispielsweise werden die Messdaten mittels der Übertragungseinrichtung 114 per SMS auf ein Mobiltelefon übertragen.

Auf der Hauptplatine 115 ist eine Bedienkonsole 116 zur Eingabe von Befehlen für die Steuer- und Auswerteeinrichtung 112 angeordnet. Derartige Befehle umfassen beispielsweise die Programmierung des Messablaufs und des Messzeitschemas. In der Fig. 1 weist die Bedienkonsole 116 ein Display 117 zum Anzeigen der Befehle, der Mess- und Kenndaten und/oder des Betriebszustands sowie ein Tastenfeld 118 zur Eingabe von Daten (Messablauf, Messzeitschema, Datenübermittlungszeitschema, Telefonnummer, Pincode der SIM-Karte etc.) auf. Anstelle des Displays 117 und des Tastenfelds 118 kann die Bedienkonsole 116 einen Touchscreen aufweisen.

Die oben beschriebenen Bauteile der Vorrichtung 100 sind in einem wasserdichten Gehäuse 119, welches z.B. aus PVC (Polyvinylchlorid) hergestellt ist, angeordnet. Das wasserdichte Gehäuse 119 weist eine abgedichtete Zuleitungsöffnung 119a und eine abgedichtete Ableitungsöffnung 119b auf, durch welche sich die Zuleitung 101 bzw. die Ableitung 105, aus dem wasserdichten Gehäuse 119 heraus in den Wasserkörper 111 der Wasserleitung 102 erstrecken.

Die Vorrichtung 100 kann beispielsweise mit Hilfe von Kabelbindern oder dergleichen an vorhandenen Bauelementen der wasserwirtschaftlichen Anlage fixiert werden.

Zur Energieversorgung ist der Vorrichtung 100 ein Energieversorgungsmodul 113, z.B. in Form von handelsüblichen Trockenbatterien, zugeordnet. Um einen Leistungsabfall oder ein Ausfall des Energieversorgungsmoduls 113, bei welchen eine Funktionssicherheit des Mess-und Reinigungsablaufs nicht mehr gewährleistet werden kann, zu erkennen ist der Vorrichtung 100 zur Batteriestandskontrolle ferner eine Alarmeinrichtung zugeordnet, welche bei geringer oder einem Ausfall der Energieversorgung ein Alarmsignal auslöst. Die Alarmeinrichtung kann Bestandteil der Steuer- und Auswerteeinrichtung 112 oder ein eigenes Bauteil sein. Funktionsstörungen in der Energieversorgung können dem Betreiber der wasserwirtschaftlichen Anlage mittels eines akustischen Signals, einer Anzeige am Display 117 oder durch die Übertragung einer Information mittels der Übertragungseinrichtung 114 an den externen Empfänger, z.B. in Form eines SMS an ein Mobiltelefon, mitgeteilt werden.

Darüber hinaus kann mittels eines nicht dargestellten Zählers (Counter), der die Messzyklen zählt, der Füllstand der Reinigungsflüssigkeit überwacht werden, wobei in Analogie zur Batteriestandskontrolle ein Alarmsignal wie oben beschrieben übermittelt wird, wenn der Füllstand eine vorgegebene Grenze unterschreitet.

Der Vorrichtung 100 kann ferner ein Temperatursensor 120 zugeordnet sein, der sich mit Vorteil in der Nähe der Messkammer 121 befindet und mit der Steuer- und Auswerteeinrichtung 112 in Signalverbindung steht. Temperaturabweichungen, die über einen vorgebbaren Temperaturbereich hinausgehen, können ebenfalls als Alarmsignal wie oben beschrieben übermittelt werden.

Weitere Signalleitungen (e), (f), (g) und (l) zwischen der Übertragungseinheit 114, dem Energieversorgungsmodul 113, dem Temperatursensor 120 und der Hauptplatine 115 bzw. zwischen der Steuer- und Auswerteeinrichtung 112 und der Bedienkonsole 116 sind ebenfalls in der Fig. 1 dargestellt.

Fig. 2 zeigt eine vergrößerte schematische Schnittdarstellung der optischen Messeinrichtung 104 aus der Fig. 1 (in Aufsicht). Außerdem ist noch die Steuer- und Auswerteeinrichtung 112 dargestellt. Die optische Messeinrichtung 104 umfasst die bereits erwähnte Messkammer 121, die mit der zu messenden Wasserprobe bzw. mit Reinigungsflüssigkeit wie oben beschrieben befüllbar ist. Bei der gezeigten Messkammer 121 handelt es sich um eine für UV-Licht durchlässige Durchflussküvette, die beispielsweise aus Quarzglas oder Kunststoff gefertigt ist. Die optische Messeinrichtung 104 umfasst ferner eine optische Messanordnung, die aus mehreren optischen Sensoren besteht, die außerhalb der Messkammer 121 an dieser angeordnet sind.

Der erste optische Sensor 104a weist als Lichtquelle eine gesteuerte UV-LED 122 mit einer Spitzenintensität von 254 nm und eine erste Photodiode 123 zum Messen der Lichtintensität, die durch die in der Messkammer 104 befindliche Flüssigkeit (Wasserprobe, Referenzflüssigkeit) transmittiert wird, auf. In Analogie zum ersten optischen Sensor 104a weist der zweite optische Sensor 104b als Lichtquelle eine gesteuerte LED 125 mit einer Spitzenintensität von 550 nm (sichtbares Licht im grünen Bereich) und eine zweite Photodiode 126 zum Messen der Lichtintensität, die durch die in der Messkammer 104 befindliche Flüssigkeit (Wasserprobe, Referenzflüssigkeit) transmittiert wird, auf. Eine optionale Weiterbildung der Messeinrichtung 104 umfasst ferner einen dritten optischen Sensor 104a, der als Lichtquelle eine gesteuerte LED 128 mit einer Spitzenintensität bei 436 nm (sichtbares Licht im blauen Bereich) und eine dritte Photodiode 129 zum Messen der Lichtintensität, die durch die in der Messkammer 104 befindliche Flüssigkeit (Wasserprobe, Referenzflüssigkeit) transmittiert wird, aufweist. Die Messpfade, die zwischen den jeweiligen LEDs 122, 125, 128 und den Photodioden 123, 126 und 129 verlaufen, sind mit Pfeilen gekennzeichnet. Die Messkammer 121 und die oben beschriebenen optischen Bauteile sind im gezeigten Beispiel in einem Kunststoffblock (als schraffiertes Rechteck dargestellt) eingebettet, wobei die Lichtkanäle, durch welche die Messpfade verlaufen, in den Kunststoffblock eingefräst sind. Die LEDs 122, 125, 128 und die Photodioden 123, 126, 129, 131 sind von außen austauschbar. Die LEDs 122, 125 und 128 werden von der Steuer-und Auswerteeinheit 112 nach an sich bekannter Art über Signalverbindungen (h), (i) und (j) gesteuert.

Die Fig. 2 zeigt darüber hinaus eine vorteilhafte Weiterbildung der Messeinrichtung 104. Um Schwankungen der Intensität der UV-LED 122 zu kompensieren, wird deren Intensität zusätzlich mit einer Referenzphotodiode 131 gemessen. Dazu ist im Messpfad zwischen der UV-LED 122 und der Messkammer 121 ein Strahlteiler 133 in Form eines halbdurchlässigen Spiegels angeordnet, der etwa 30% des von der UV-LED 122 emittierten UV-Lichts in einen Referenzmesspfad abzweigt und der Referenzphotodiode 131 zuführt.

Alternativ dazu ist es auch möglich, Schwankungen der Intensität der UV-LED 122 zu kompensieren, indem die Schwellspannung der ersten Photodiode 123 erfasst und bei der Messdatenauswertung durch die Steuer- und Auswerteeinheit 112 berücksichtigt wird. In diesem Fall kann auf einen Strahlteiler und eine Referenzphotodiode wie oben beschrieben verzichtet werden.

Eine weitere vorteilhafte Weiterbildung der Messeinrichtung 104, die ebenfalls in Fig. 2 dargestellt ist, umfasst einen zwischen der Messkammer 121 und der ersten Photodiode 123 angeordneten Bandpassfilter 137. Mittels des Bandpassfilters 137 können Fehlmessungen, die auf eine zu große Bandbreite zurückzuführen sind, ausgeschlossen werden. Die Bandbreite ist von den eingesetzten optischen Bauelementen abhängig und ergibt sich aus der Intensitätskennlinie der ersten Lichtquelle, der Transmissionskennlinie der Messkammer und des gegebenenfalls vorhandenen Strahlteilers sowie der Empfindlichkeitskennlinie der ersten Photoelektrode.

Die von den ersten, zweiten und dritten Photodioden 123, 126 und 129 sowie von der Referenzphotodiode 131 erfassten Lichtintensitäten werden nach an sich bekannter Art in entsprechende elektrische Signale umgewandelt, welche anschließend in Messverstärkern 124, 127, 130 und 132, die den Photodioden nachgeschaltet sind, amplifiziert und über Signalverbindungen (k) der Steuer- und Auswerteeinrichtung 112 für die weitere Messdatenauswertung zugeführt werden.

Fig. 3 zeigt eine weitere vergrößerte schematische Schnittdarstellung der Messeinrichtung 104 aus der Fig. 1 (in Seitenansicht), wobei der Schnitt durch die oben in Fig. 2 beschriebenen optischen Bauteile des ersten optischen Sensors 104a (UV-LED 122, Strahlteiler 133, erste Photodiode 123, Messverstärker 124, Lichtkanal 134) und die Messkammer 121 führt.

Aus der Fig. 3 sind ferner die Anschlüsse 135,136 für die Zuleitung 101 der Flüssigkeit in die Messkammer 121 und für die Ableitung 105 der Flüssigkeit aus der Messkammer 121 erkennbar.

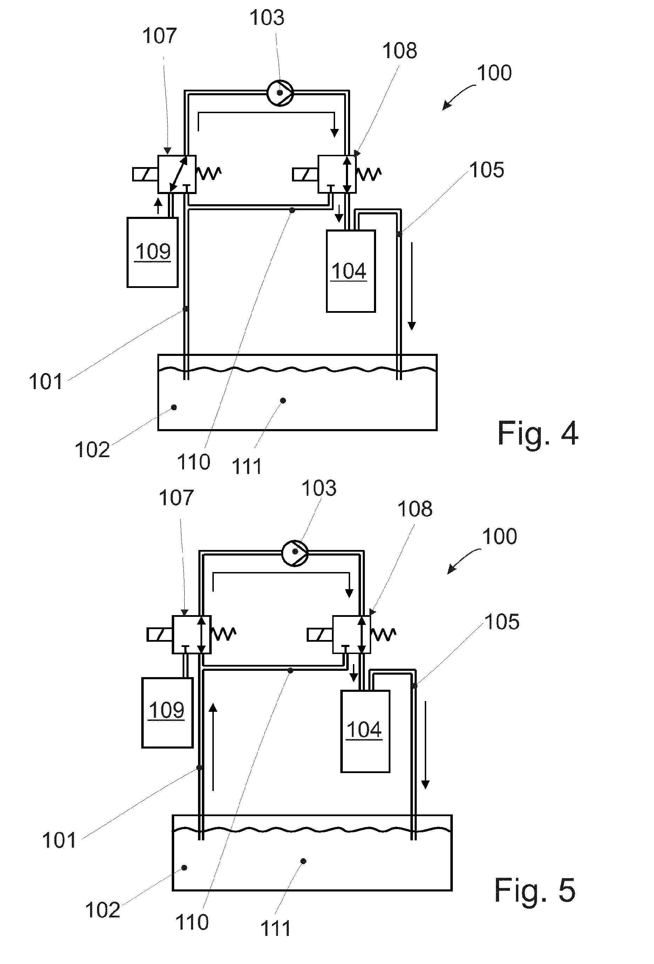

Fig. 4 bis 7 illustrieren die Schritte eines Messablaufs anhand der Darstellung der wesentlichen Vorrichtungskomponenten aus der Fig. 1, wobei die Fließrichtung der durch die Leitungen 101, 105, 110 und die Messkammer 121 der Vorrichtung 100 strömenden Wasserprobe (im vorliegende Beispiel handelt es sich um Abwasser) bzw. Reinigungsflüssigkeit sowie die Ventilstellungen des Ansaugventils 107 und des Rückspülventils 108 mit Hilfe von Pfeilen dargestellt sind.

In einem ersten, in der Fig. 4 dargestellten Schritt wird die Messkammer 121 mit der Reinigungsflüssigkeit vorgespült. Dafür wird mittels der Membranpumpe 103 die Reinigungsflüssigkeit aus dem Reinigungsbehälter 109 bei entsprechenden Ventilstellungen des Ansaugventils 107 und des Rückspülventils 108 über die Zuleitung 101 in die Messkammer 121 der Messeinrichtung 104 und von dort über die Ableitung 105 in die Wasserleitung 102 geleitet. Sobald die Messkammer 121 mit der Reinigungsflüssigkeit befüllt ist, werden zum Kalibrieren mittels der ersten, zweiten und dritten optischen Sensoren 104a, 104b, 104c Referenzmessungen (Nullmessungen) bei den Wellenlängen 254 nm, 550 nm und gegebenenfalls bei 436 nm durchgeführt. Durch die Referenzmessung mit der Reinigungsflüssigkeit können auch Temperatureinflüsse kompensiert werden. Die Reinigungsflüssigkeit dient somit gleichzeitig als Referenzflüssigkeit, wobei es sich hierbei vorzugsweise um optisch reines Wasser handelt. Beispielsweise kann das optisch reine Wasser eine der DIN-Norm Nr. 38404-3/Juli 2005 entsprechende Qualität nach DIN ISO 3639 aufweisen.

Im darauffolgenden Schritt des Messablaufs, der in der Fig. 5 dargestellt ist, wird nach Änderung der Ventilstellung des Ansaugventils 107 die Abwasserprobe aus dem Wasserkörper 111 der Wasserleitung 102 in die Messkammer 121 (siehe Detailansicht in Fig. 2 und 3) der Messeinrichtung 104 und von dort über die Ableitung 105 wieder zurück in die Wasserleitung 102 geleitet. Sobald die Messkammer 121 mit der Wasserprobe befüllt ist, werden in Analogie zu der oben beschriebenen Referenzmessung mittels der ersten, zweiten und dritten optischen Sensoren 104a, 104b, 104c die optischen Eigenschaften der Wasserprobe bei den Wellenlängen 254 nm, 550 nm und gegebenenfalls bei 436 nm photometrisch bestimmt.

Die Messung der optischen Eigenschaften in Reinigungsflüssigkeit bzw. in Abwasser mit dem dritten Sensor 104c bei einer Wellenlänge von 436 nm zur Bestimmung des DOC ist optional. Handelt es sich bei der zu untersuchenden Wasserprobe um Trink- oder Grundwasser, dann kann anhand des DOC auch Rückschluss auf die Färbung/Huminstoffbelastung gezogen werden.

Wie in Fig. 2 beschrieben, wird sowohl bei der Referenzmessung in Reinigungsflüssigkeit als auch bei der Messung der Wasserprobe (Abwasser) die Referenzintensität der UV-LED 122 des ersten optischen Sensors zusätzlich mit der Referenzphotodiode 131 gemessen, um Schwankungen der Intensität der UV-LED 122 zu kompensieren.

Die Referenzmessung in Reinigungsflüssigkeit und die Messung der Abwasserprobe erfolgt üblicherweise durch zyklisches Abtasten der einzelnen Photodioden. Beispielsweise werden insgesamt 256 Zyklen durchgeführt und die Messwerte gemittelt. Die Messdauer beträgt dabei insgesamt etwa 1 Sekunde. Das Resultat ist ein ganzzahliger Wert von 0 - 1000, welcher die Lichtintensität repräsentiert.

Folgende Messwerte der Lichtintensität [I] werden demnach mittels der ersten, zweiten und dritten Sensoren 104a, 104, 104c erhalten:

254 nm (UV) Intensität bei Reinigungsflüssigkeit: $I_{UvSpuMes}$
245 nm (UV) Referenzintensität bei Reinigungsflüssigkeit: $I_{UvSpuRef}$
254 nm (UV) Intensität bei Abwasser: $I_{UvAbwMes}$
254 nm (UV) Referenzintensität bei Abwasser: $I_{UvAbwRef}$
550 nm (Grün) Intensität bei Reinigungsflüssigkeit: $I_{GrSpu}$
550 nm (Grün) Intensität bei Abwasser: $I_{GrAbw}$
436 nm (Blau) Intensität bei Reinigungsflüssigkeit: $I_{BlSpu}$
436 nm (Blau) Intensität bei Abwasser: $I_{BlAbw}$

Der Begriff "Referenzintensität" bezieht sich dabei auf die Lichtintensität, die mit der Referenzphotoelektrode 131 gemessen wird.

Folgende Berechnungen werden anhand der gemessenen Lichtintensitäten mittels der Steuer- und Auswerteeinrichtung 112 für Abwasser durchgeführt:

$$SSK_{254}[1/m] = f/d_1 * \{[Log(I_{UvSpuMes})-Log(I_{UvSpuRef})]+$$
$$[Log(I_{UvAbwRef})-Log(I_{UvAbwMes})]\}$$

$$SSK_{550}[1/m] = f/d_2 * [Log(I_{GrSpu}) - Log(I_{GrAbw})]$$

$$SSK_{436}[1/m] = f/d_2 * [Log(I_{BlSpu}) - Log(I_{BlAbw})]$$

($SSK_{254}$ = spektraler Streuungskoeffizient bei 254 nm)
($SSK_{550}$ = spektraler Streuungskoeffizient bei 550 nm)
($SSK_{436}$ = spektraler Streuungskoeffizient bei 436 nm)

(f = der Faktor, um den spektralen Absorptionskoeffizienten in $m^{-1}$ zu erhalten, typischerweise ist f = 1000)

($d_1$, $d_2$ = optische Weglängen der Messkammer 121 in mm. Je nach Konstruktion der Messkammer 121 können $d_1$ und $d_2$ gleich oder unterschiedlich sein. Wie in Fig. 2 gezeigt ist aufgrund der besseren zeichnerischen Darstellbarkeit die optische Weglänge $d_1$ für den ersten Sensor 104a (Messung des $SSK_{254}$) länger als die optische Weglänge $d_2$ für den zweiten und den dritten optischen Sensor 104b, 104c (Messung $SSK_{550}$ bzw. $SSK_{436}$). In der Praxis kann es z.B. bei stark getrübten Wasserproben von Vorteil sein, wenn die optische Weglänge für den ersten Sensor 104a kürzer gewählt ist.)

$$SAK_{254}\ [1/m]\ = SSK_{254} - SSK_{550}$$

$$SAK_{436}\ [1/m]\ = SSK_{436} - SSK_{550}$$

($SAK_{254}$ = spektraler Absorptionskoeffizient bei 254 nm)
($SAK_{436}$ = spektraler Absorptionskoeffizient bei 436 nm)

$$CSB\ \ [mg/l] = 2{,}39 * SAK_{254} - 19$$

$$BSB_5\ \ [mg/l] = 0{,}86 * SAK_{254} - 26{,}42$$

(CSB = chemischer Sauerstoffbedarf)
($BSB_5$ = biologischer Sauerstoffbedarf nach 5 Stunden)

Die Vorrichtung 100 ist aufgrund der oben beschriebenen Sensoren 104a, 104b, 104c für die photometrische Überwachung von Abwasserparametern wie den CSB oder den BSB5 ausgerichtet. Sie kann jedoch auch zur photometrischen Überwachung von Trinkwasserparametern eingesetzt werden, wobei bei Trinkwasser die Messung bei einer Wellenlänge von 550 nm entfallen kann, da Trinkwasser üblicherweise keine ungelösten Bestandteile aufweist. Durch entsprechende Programmierung des Messablaufs können in Abhängigkeit der zu untersuchenden Wasserprobe (z.B. Abwasser, Trinkwasser) die entsprechenden Sensoren 104a, 104b, 104c unabhängig voneinander von der Steuer- und Auswerteeinrichtung 112 gesteuert/aktiviert werden.

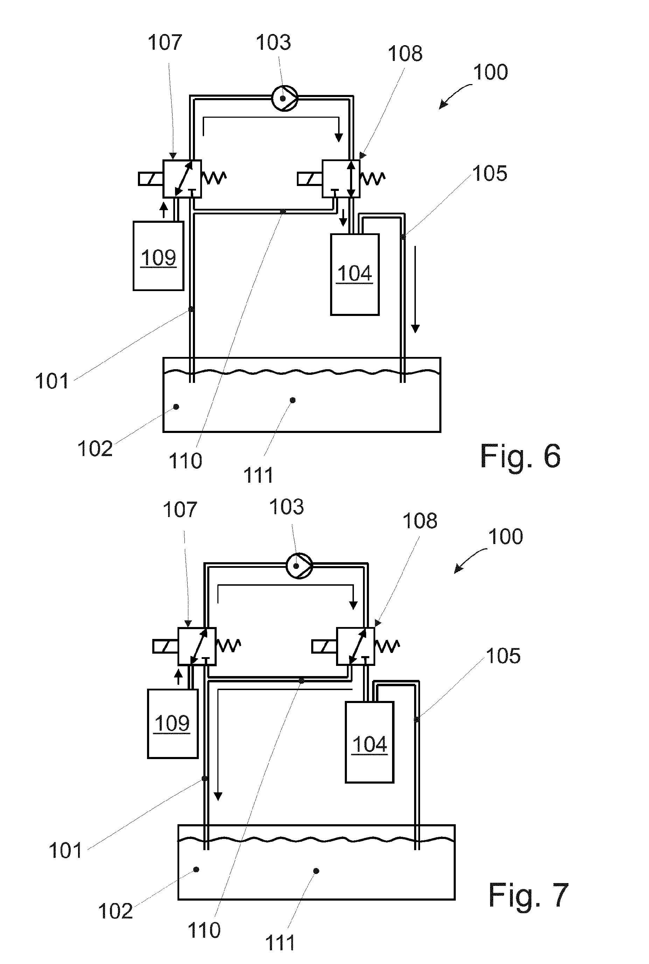

Im nächsten Schritt, der in der Fig. 6 dargestellt ist, werden die Messkammer 112 der Messeinrichtung 104 und die Leitungen 101, 105 mit Reinigungsflüssigkeit gespült und gereinigt, um Verunreinigungen zu beseitigen und einer Biofilmbildung entgegenzuwirken. Die Ventilstellungen des Ansaugventils 107 und des Rückspülventils 108 entsprechen dabei jenen aus der Fig. 4.

Um denjenigen Abschnitt der Zuleitung 101, der stromauf des Ansaugventils 107 zwischen dem Ansaugventil 107 und dem Wasserkörper 111 liegt, zu reinigen, wird in einem weiteren Schritt, der in der Fig. 7 dargestellt ist, die Reinigungsflüssigkeit durch entsprechende Ventilstellung des Rückspülventils 108 in die Rückspülleitung 110 und weiter in die Zuleitung 101 geleitet. Derjenige Abschnitt der Zuleitung 101, der stromauf des Ansaugventils 107, d.h. im Wesentlichen zwischen Ansaugventil 107 und Wasserleitung 102, liegt, wird daher durch Spülen mit Reinigungsflüssigkeit in eine Fließrichtung entgegen der Fließrichtung der entnommenen Wasserprobe (vergleiche Fig. 5) gereinigt.

Im Anschluss an den in Fig. 7 dargestellten Reinigungsschritt wird die Messeinrichtung in Ruhestellung versetzt (Membranpumpe 103 aus, Ventile 107, 108 stromlos). Die erhaltenen Messdaten werden wie oben beschrieben mittels der Steuer- und Auswerteeinrichtung 112 ausgewertet und mit Hilfe der Übertragungseinrichtung 114 (GSM-Modul) an einen externen Empfänger (SMS an Mobiltelefon) übermittelt. Um beispielsweise eine SMS an ein Mobiltelefon zu übermitteln, werden die Telefonnummer des Mobiltelefons sowie der PINCode der SIM-Karte der Übertragungseinrichtung 114 eingegeben. In der SMS werden der Status der Messung, SAK254 Wert, CSB Wert, BSB Wert, SSK550 Wert, Temperatur und Batteriespannung zum Beispiel wie folgt übermittelt:

Tag: FR
Messung Ok
VS: 0x

SAK: 26<1/m>
CSB: 43<mg/l>
BSB: 0<mg/l>
SSK550: 0<mg/l>
Temp 21 <C>
VBat: 11,8 <V>

Das Zeitschema des in den Fig. 4 bis 7 beschriebenen Messablaufs kann daher beispielsweise wie folgt aussehen:

1) 8 Sekunden Vorspülen der Messkammer 121 mit Reinigungsflüssigkeit (optisch reines Wasser).
2) 20 Sekunden warten und Referenzmessung in Reinigungsflüssigkeit zur Kalibrierung.
3) 30 Sekunden lang Ansaugen von Abwasser durch die Messkammer 121.
4) 20 Sekunden lang warten und Messung im Abwasser durchführen.
5) 8 Sekunden Spülen der Messkammer 121 mit Reinigungsflüssigkeit zur Reinigung.
6) 8 Sekunden Spülen des stromauf des Ansaugventils 107 liegenden Abschnitts der Zuleitung 101 mit Reinigungsflüssigkeit durch Rückleiten der Reinigungsflüssigkeit über das Rückspülventil 108 und die Rückspülleitung 110 in die Zuleitung 101.
7) Ruhestellung (Pumpe 103 aus, Ventile 107,108 stromlos) und Auswerten und Schicken der Messdaten per SMS an ein Mobiltelefon.

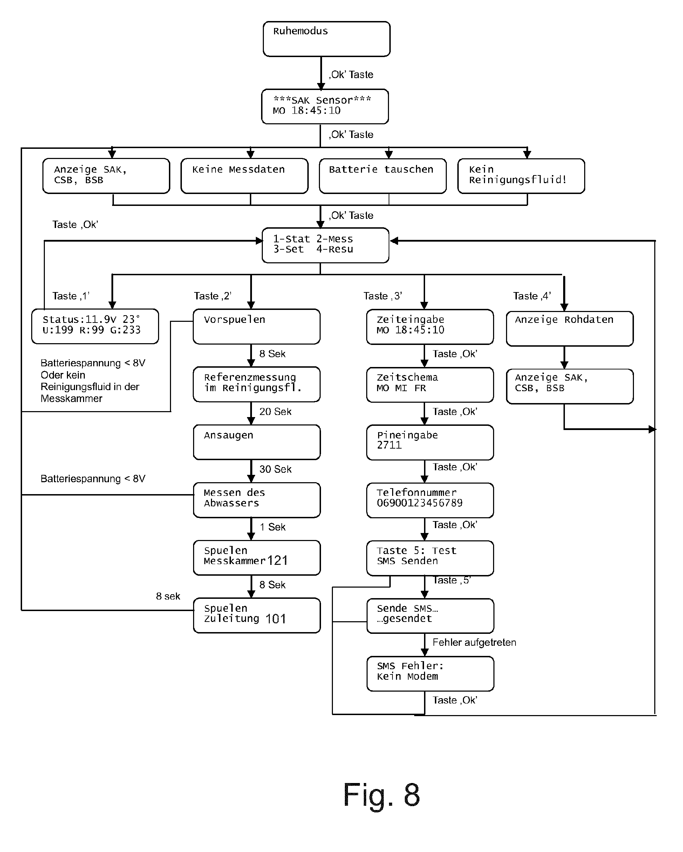

Fig. 8 zeigt ein Ablaufdiagramm des in den Fig. 4 bis 7 beschriebenen Messablaufs samt Menüstruktur für die Befehlseingabe und unter Bezugnahme auf das in Fig. 1 dargestellte Tastenfeld 118.

Der in den Fig. 4 bis 8 beschriebene Messablauf kann über ein programmiertes Zeitschema, das durch Eingabe von Befehlen in der Bedienkonsole 116 programmierbar ist, zu gewünschten Tageszeiten aktiviert werden. Üblicherweise werden die Messungen in periodischen Zeitabständen zu gewissen Uhrzeiten und/oder an gewissen Tagen durchgeführt. Außerhalb des Zeitschemas kann eine Sofortmessung durchgeführt werden.

Zwischen den einzelnen Messungen befindet sich die Vorrichtung 100 vorzugsweise im Stromsparmodus. Dabei wird die Stromversorgung der Pumpe 103, der Ventile 107, 108, der Anzeige am Display 117 und der Peripheriekomponenten (GSM Modem, Messverstärker etc.) abgeschaltet.

Die hierin gezeigten Figuren sind zum Verständnis des Messprinzips ausgeführt und nicht als exakte Konstruktionszeichnungen gedacht.

Das oben beschriebene Ausführungsbeispiel samt seinen vorteilhaften Weiterbildungen ist nur eines unter vielen und nicht als einschränkend zu betrachten.

**Patentansprüche**

1. Vorrichtung (100) zur Überwachung von Parametern einer wässrigen Flüssigkeit, insbesondere Abwasser, aufweisend:

eine Messkammer (121) mit einer Zuleitung (101) für die zu untersuchende Flüssigkeit und einer Ableitung (105), eine optische Messanordnung mit zumindest einem optischen Sensor (104a, 104b) zur Messung zumindest einer optischen Eigenschaft der Flüssigkeit, und
eine Steuer- und Auswerteeinrichtung (112), wobei
die optische Messanordnung einen ersten optischen Sensor (104a) und einen zweiten optischen Sensor (104b) aufweist, wobei der erste optische Sensor (104a) eine erste Lichtquelle (122), die UV-Licht emittiert, einen ersten Photodetektor (124) und einen zwischen der ersten Lichtquelle (122) und dem ersten Photodetektor (124) verlaufenden ersten Messpfad aufweist, wobei der zweite optische Sensor (104b) eine zweite Lichtquelle (125), die sichtbares Licht emittiert, einen zweiten Photodetektor (126) und einen zwischen der zweiten Lichtquelle (125) und dem zweiten Photodetektor (126) verlaufenden zweiten Messpfad aufweist, wobei der erste und der zweite Messpfad durch die Messkammer (121) und die darin befindliche Flüssigkeit verlaufen,
die Zuleitung (101) oder die Ableitung (105) zumindest ein Ventilmittel (107) aufweist, durch welches zumindest ein Reinigungsfluid in die Messkammer (121) zuführbar ist,
die Vorrichtung (100) zumindest ein Pumpmittel (103) zum Leiten der Flüssigkeit bzw. des zumindest einen Reinigungsfluids durch die Messkammer (121) aufweist, und
die Steuer- und Auswerteeinrichtung (112) zur programmierbaren Steuerung zumindest der Pump- und Ventilmittel (103, 107, 108) und zum Auswerten der mittels der optischen Messanordnung ermittelten Messwerte eingerichtet ist,

**dadurch gekennzeichnet, dass**

die Steuer- und Auswerteeinrichtung (112) dazu eingerichtet ist, aufgrund der mit Reinigungsfluid befüllten Messkammer (121) Referenzmessungen durchzuführen.

2. Vorrichtung nach Anspruch 1, **dadurch gekennzeichnet, dass** die erste Lichtquelle (122) UV-Licht mit einer Wellenlänge von 254 nm und die zweite Lichtquelle (125) sichtbares Licht mit einer Wellenlänge von 550 nm emittiert.

3. Vorrichtung nach Anspruch 1 oder 2, **dadurch gekennzeichnet, dass** die optische Messanordnung einen dritten optischen Sensor (104c) aufweist, der eine dritte Lichtquelle (128), die sichtbares Licht emittiert, einen dritten Photodetektor (129) und einen zwischen der dritten Lichtquelle (128) und dem dritten Photodetektor (129) verlaufenden dritten Messpfad aufweist, wobei der dritte Messpfad durch die Messkammer (121) und die darin befindliche Flüssigkeit verläuft.

4. Vorrichtung nach Anspruch 2 und 3, **dadurch gekennzeichnet, dass** die dritte Lichtquelle (128) sichtbares Licht mit einer Wellenlänge von 436 nm emittiert.

5. Vorrichtung nach einem der Ansprüche 1 bis 4, **dadurch gekennzeichnet, dass** die erste, die zweite und die dritte Lichtquelle (122, 125, 128) eine LED (Light Emitting Diode) aufweisen.

6. Vorrichtung nach einem der Ansprüche 1 bis 5, **dadurch gekennzeichnet, dass** zwischen der ersten Lichtquelle (122) und der Messkammer (121) ein Strahlteiler (133) angeordnet ist, welcher einen Anteil des von der ersten Lichtquelle (122) emittierten UV-Lichts in einen Referenzmesspfad abzweigt und einem Referenzphotodetektor (131) zuführt.

7. Vorrichtung nach einem der Ansprüche 1 bis 5, **dadurch gekennzeichnet, dass** die Steuer- und Auswerteeinheit (112) dazu eingerichtet ist, die Schwellspannung des ersten Photodetektors (123) zu erfassen und bei der Auswertung der Messwerte zu berücksichtigen.

8. Vorrichtung nach einem der Ansprüche 1 bis 7, **dadurch gekennzeichnet, dass** im ersten Messpfad an einer Position zwischen der ersten Lichtquelle (122) und dem ersten Photodetektor (123) ein Bandpassfilter (137) angeordnet ist.

9. Vorrichtung nach einem der Ansprüche 1 bis 8, **gekennzeichnet durch** eine Übertragungseinheit (114), insbesondere ein GSM-Modul, zum Übertragen der ermittelten und/oder ausgewerteten Messwerte an einen externen Empfänger.

10. Vorrichtung nach einem der Ansprüche 1 bis 9, **gekennzeichnet durch** eine Bedienkonsole (116) zur Eingabe von Befehlen für die Steuer- und Auswerteeinrichtung (112).

11. Vorrichtung nach einem der Ansprüche 1 bis 10, **dadurch gekennzeichnet, dass** die Vorrichtung (100) zur Energieversorgung ein austauschbares Energieversorgungsmodul (113) aufweist.

12. Vorrichtung nach Anspruch 11, **dadurch gekennzeichnet, dass** der Vorrichtung (100) eine Alarmeinrichtung zugeordnet ist, welche bei geringer oder einem Ausfall der Energieversorgung ein Alarmsignal auslöst.

13. Vorrichtung nach einem der Ansprüche 1 bis 12, **dadurch gekennzeichnet, dass** das zumindest eine Ventilmittel (107) in der Zuleitung (101) angeordnet ist und wobei in der Zuleitung (101) an einer Position zwischen dem zumindest einen Ventilmittel (107) und der Messkammer (121) ein Rückspülventil (108) angeordnet ist, wobei sich vom Rückspülventil eine Rückspülleitung (110) zur Zuleitung (101) erstreckt und wobei die Rückspülleitung (110) an einer Position stromauf des zumindest einen Ventilmittels (107) in die Zuleitung (101) mündet.

14. Vorrichtung nach einem der Ansprüche 1 bis 13, **gekennzeichnet durch** ein wasserdichtes Gehäuse (119), in welchem die Vorrichtung (100) angeordnet ist, wobei das Gehäuse (119) Zu- und Ableitungsöffnungen (119a, 119b) aufweist, **durch** welche sich die Zu- und Ableitungen (101, 105) erstrecken.

15. Verfahren zum Überwachen von Parametern einer wässrigen Flüssigkeit, insbesondere Abwasser, unter Verwendung einer Vorrichtung (100) nach einem der Ansprüche 1 bis 14, umfassend die folgenden Schritte:

a) Spülen der Messkammer (121) mit der wässrigen Flüssigkeit,

b) Bestimmen der optischen Eigenschaften der wässrigen Flüssigkeit mittels des ersten optischen Sensors (104a, 104b), und gegebenenfalls mittels des zweiten und/oder dritten optischen Sensors (104c),

c) Spülen der Messkammer (121) mit zumindest einem Reinigungsfluid,

d) Auswerten der mittels der optischen Sensoren (104a, 104b, 104c) erhaltenen Messwerte mittels der Steuer- und Auswerteeinrichtung (112), und

e) Übermitteln der ausgewerteten Messwerte mittels einer Übertragungseinheit (114), insbesondere eines GSM-Moduls, an einen externen Empfänger,

wobei das Verfahren **dadurch gekennzeichnet ist, dass**
vor Schritt a) mittels der ersten und zweiten optischen Sensoren (104a, 104b), und gegebenenfalls mittels des dritten optischen Sensors (104c), zumindest eine Referenzmessung durch Bestimmen der optischen Eigenschaften des sich in der Messkammer (121) befindlichen Reinigungsfluids durchgeführt wird.

16. Verfahren nach Anspruch 15, **dadurch gekennzeichnet, dass** anschließend an Schritt c) die Zuleitung (101) der Messkammer (121) mittels zumindest eines Reinigungsfluids rückgespült wird.

17. Verfahren nach einem der Ansprüche 15 oder 16, **dadurch gekennzeichnet, dass** in Schritt d) die Schwellspannung des ersten Photodetektors (123) bei der Auswertung der Messwerte berücksichtigt wird.

18. Verfahren nach einem der Ansprüche 15 bis 17, **dadurch gekennzeichnet, dass** die Messungen mittels des ersten optischen Sensors (104a) bei einer Wellenlänge von 254 nm, mittels des zweiten optischen Sensors (104b) bei einer Wellenlänge von 550 nm und gegebenenfalls mittels des dritten optischen Sensors (104c) bei einer Wellenlänge von 436 nm durchgeführt werden.

100

116    115         107      103         108

(a)

117    (g)         (b)

112    (c)

109                    (d)         104

(e)         (f)        (l)

113         114        120

118

119

101
119a
101a
102        111a         105
111    110              119b

# Fig. 1

108

Fig. 2

Fig. 3

Fig. 4

Fig. 5

Fig. 6

Fig. 7

```
                            ┌─────────────────┐
                            │   Ruhemodus     │
                            └────────┬────────┘
                                     │ ‚Ok' Taste
                            ┌────────▼────────┐
                            │ ***SAK Sensor***│
                            │ MO 18:45:10     │
                            └────────┬────────┘
                                     │ ‚Ok' Taste
        ┌────────────────┬───────────┼──────────────┬─────────────────┐
┌───────▼─────────┐ ┌────▼──────────┐ ┌──────▼──────────┐ ┌────▼──────────────┐
│ Anzeige SAK,    │ │ Keine Messdaten│ │ Batterie tauschen│ │ Kein              │
│ CSB, BSB        │ │               │ │                 │ │ Reinigungsfluid!  │
└───────┬─────────┘ └───────────────┘ └─────────────────┘ └───────────────────┘
        │                              │ ‚Ok' Taste
```

Taste ‚Ok'

```
                            ┌─────────────────┐
                            │ 1-Stat 2-Mess   │
                            │ 3-Set  4-Resu   │
                            └─────────────────┘
```

Taste ‚1'          Taste ‚2'          Taste ‚3'          Taste ‚4'

```
┌─────────────────┐ ┌─────────────────┐ ┌─────────────────┐ ┌─────────────────┐
│ Status:11.9v 23°│ │ Vorspuelen      │ │ Zeiteingabe     │ │ Anzeige Rohdaten│
│ U:199 R:99 G:233│ │                 │ │ MO 18:45:10     │ │                 │
└─────────────────┘ └────────┬────────┘ └────────┬────────┘ └────────┬────────┘
                        8 Sek │           Taste ‚Ok'│                  │
Batteriespannung < 8V   ┌─────▼───────────┐ ┌──────▼──────────┐ ┌──────▼──────────┐
Oder kein               │ Referenzmessung │ │ Zeitschema      │ │ Anzeige SAK,    │
Reinigungsfluid in der  │ im Reinigungsfl.│ │ MO MI FR        │ │ CSB, BSB        │
Messkammer              └────────┬────────┘ └────────┬────────┘ └─────────────────┘
                          20 Sek │           Taste ‚Ok'│
                        ┌────────▼────────┐ ┌──────▼──────────┐
                        │ Ansaugen        │ │ Pineingabe      │
                        │                 │ │ 2711            │
                        └────────┬────────┘ └────────┬────────┘
                          30 Sek │           Taste ‚Ok'│
Batteriespannung < 8V   ┌────────▼────────┐ ┌──────▼──────────┐
                        │ Messen des      │ │ Telefonnummer   │
                        │ Abwassers       │ │ 06900123456789  │
                        └────────┬────────┘ └────────┬────────┘
                           1 Sek │           Taste ‚Ok'│
                        ┌────────▼────────┐ ┌──────▼──────────┐
                        │ Spuelen         │ │ Taste 5: Test   │
                        │ Messkammer 121  │ │ SMS Senden      │
                        └────────┬────────┘ └────────┬────────┘
                           8 Sek │            Taste ‚5'│
              8 sek     ┌────────▼────────┐ ┌──────▼──────────┐
                        │ Spuelen         │ │ Sende SMS...    │
                        │ Zuleitung 101   │ │ ...gesendet     │
                        └─────────────────┘ └────────┬────────┘
                                             Fehler aufgetreten
                                            ┌──────▼──────────┐
                                            │ SMS Fehler:     │
                                            │ Kein Modem      │
                                            └─────────────────┘
                                                Taste ‚Ok'
```

Fig. 8

Europäisches
Patentamt

European
Patent Office

Office européen
des brevets

# EUROPÄISCHER RECHERCHENBERICHT

Nummer der Anmeldung

EP 13 19 6915

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 92/16828 A2 (WELSH WATER ENTERPRISES LTD [GB]) 1. Oktober 1992 (1992-10-01) * Seite 1, Zeilen 2-13 * * Seite 2, Zeilen 9-25 * * Seite 3, Zeilen 17-21 * * Seite 4, Zeile 17 - Seite 5, Zeile 5 * * Seite 5, Zeile 27 - Seite 6, Zeile 7 * * Seite 8, Zeilen 14-18 * * Seite 9, Zeilen 3-10 * * Seite 11, Zeilen 9,10,20-24 * * Seite 12, Zeilen 1-11 * * Seite 13, Zeilen 12-21 * * Seite 14, Zeilen 16-19 * * Seite 16, Zeile 26 - Seite 17, Zeile 4 * * Seite 17, Zeilen 13-17 * * Seite 19, Zeilen 18-21 * ----- | 1-18 | INV. G01N21/85 G01N33/18 G01N21/15 G01N21/27 |
| A | EP 0 985 920 A1 (NAPHTACHIMIE SA [FR]) 15. März 2000 (2000-03-15) * Absätze [0006], [0009], [0011], [0013], [0015], [0020], [0021], [0024], [0031], [0057] - [0060] * ----- | 1-18 | |
| A | WO 87/03091 A1 (CONSILIUM MARINE AB [SE]) 21. Mai 1987 (1987-05-21) * Seite 5, Zeilen 15-24 * * Seite 9, Zeilen 27-31 * * Seite 12, Zeilen 21-37 * * Abbildung 5 * ----- | 1,15 | RECHERCHIERTE SACHGEBIETE (IPC) G01N C02F |

Der vorliegende Recherchenbericht wurde für alle Patentansprüche erstellt

| Recherchenort | Abschlußdatum der Recherche | Prüfer |
|---|---|---|
| Berlin | 14. März 2014 | Navas Montero, E |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

....................................................................

& : Mitglied der gleichen Patentfamilie, übereinstimmendes
    Dokument

EPO FORM 1503 03.82 (P04C03)

**EP 2 746 751 A1**

**ANHANG ZUM EUROPÄISCHEN RECHERCHENBERICHT
ÜBER DIE EUROPÄISCHE PATENTANMELDUNG NR.**  EP 13 19 6915

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-03-2014

| Im Recherchenbericht angeführtes Patentdokument | Datum der Veröffentlichung | Mitglied(er) der Patentfamilie | Datum der Veröffentlichung |
|---|---|---|---|
| WO 9216828 A2 | 01-10-1992 | AU 660305 B2 | 22-06-1995 |
| | | AU 1543992 A | 21-10-1992 |
| | | CA 2106472 A1 | 20-09-1992 |
| | | CZ 9301929 A3 | 19-10-1994 |
| | | DE 69227764 D1 | 14-01-1999 |
| | | DE 69227764 T2 | 01-07-1999 |
| | | EP 0576501 A1 | 05-01-1994 |
| | | ES 2127215 T3 | 16-04-1999 |
| | | FI 934096 A | 17-09-1993 |
| | | IE 920884 A1 | 23-09-1992 |
| | | JP H06508431 A | 22-09-1994 |
| | | NZ 242027 A | 26-07-1995 |
| | | US 5420432 A | 30-05-1995 |
| | | WO 9216828 A2 | 01-10-1992 |
| EP 0985920 A1 | 15-03-2000 | KEINE | |
| WO 8703091 A1 | 21-05-1987 | SE 450791 B | 27-07-1987 |
| | | WO 8703091 A1 | 21-05-1987 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009028254 A1
- GB 2256043 A
- EP 0724718 A
- DE 10228929 A1
- DE 102008028058 A1
- DE 102004063720
- DE 69227764 T2
- DE 69107551 T2
- EP 1962089 B1